(19) Europäisches Patentamt

European Patent Office

Office européen des brevets

(11) **EP 1 661 884 A1**

(12) **EUROPEAN PATENT APPLICATION**
published in accordance with Art. 158(3) EPC

(43) Date of publication:
**31.05.2006 Bulletin 2006/22**

(51) Int Cl.:
***C07C 239/20*** (1990.01)

(21) Application number: **03818543.5**

(22) Date of filing: **29.08.2003**

(86) International application number:
**PCT/JP2003/011086**

(87) International publication number:
**WO 2005/023757 (17.03.2005 Gazette 2005/11)**

(84) Designated Contracting States:
**AT BE BG CH CY CZ DE DK EE ES FI FR GB GR
HU IE IT LI LU MC NL PT RO SE SI SK TR**
Designated Extension States:
**AL LT LV MK**

(71) Applicant: **BASF Aktiengesellschaft
67056 Ludwigshafen (DE)**

(72) Inventors:
• **IMAI, Tadashi
  Isesaki-shi, Gunma 372-0055 (JP)**
• **IMAI, Ken
  Ota-shi, Gunma 373-0806 (JP)**

(54) **PROCESS FOR PRODUCTION OF O-SUBSTITUTED HYDROXYLAMINES**

(57) [Purpose]

The purpose of the present invention is to provide a method for the production of O-substituted hydroxylamine compounds at a high yield by using industrially available starting material.

[Solution method]

Method for the production of O-substituted hydroxylamine compounds comprising a process of obtaining O-substituted hydroxylamine disulfonic acid alkali metal salts by reacting hydroxylamine disulfonic acid alkali metal salts with halides exemplified by alkylating agents (RX), and a process of hydrogenating the obtained O-substituted hydroxylamine disulfonic acid alkali metal salt.

EP 1 661 884 A1

**Description**

[Technical field of the invention]

The present invention relates to a method for the production of O-substituted hydroxylamine compounds.

[Prior art]

O-substituted hydroxylamine compounds represented by O-methyl substituted hydroxylamine compounds are compounds used as intermediates for the production of various pharmaceutical compounds and pesticides. Production methods via amineoximes or hydroxy phthalimides using hydroxyl amine as starting material represent the conventional technique. However, such conventional methods require the use of hydroxyl amine, which needs to be handled with care, as the starting material. Furthermore, the elimination of the ketone and phthalic acid moiety, respectively by hydrogenation after introducing the O-substituent is not quantitative. The disadvantages of the conventional methods were therefore the difficulty in obtaining the desired O-substituted hydroxylamine compounds in a high yield as well as the expenses of the purification process. For example, regarding the synthesis of amino-oxyacetic acid via aceto oxime disclosed in Volume 3 of Collected Organic Synthesis, the yield is only 66 to 72%.

[Problem to be solved by the invention]

The purpose of the invention is to provide a method for the production of O-substituted hydroxylamine compounds with a high yield using industrially available starting materials.

[Means for solving the problem]

Carrying out research on the production of O-substituted hydroxylamine compounds in a high yield, the inventors of the present invention came to find a novel method by using industrially available hydroxylamine disulfonic acid alkali metal salt as starting material.

The present invention concerns a method for the production of O-substituted hydroxylamine compounds comprising:

a process for obtaining O-substituted hydroxylamine disulfonic acid alkali metal salts by reacting a hydroxylamine disulfonic acid alkali metal salt with a halide according to the following formula (1):

$$(MSO_3)_2NOH + RX \quad (MSO_3)_2NOR + HX \quad \cdot \cdot \cdot (1)$$

[wherein M is an alkali metal atom; R is an alkyl group, alkenyl group, alkynyl group, aryl group, aralkyl group, carboxyl group, ester group, or ether group, where each group may have a substituent; and X is a halogen atom], and a process for hydrogenating the obtained O-substituted hydroxylamine disulfonic acid alkyl metal salt.

[Embodiment of the invention]

The present invention concerns a method for the production of O-substituted hydroxylamine compounds by using a hydroxylamine disulfonic acid alkali metal salt as starting material and reacting it with halogen compounds (e.g. alkylating agents) to carry out a conversion into O-substituted hydroxyloamine disulfonic acid alkali metal salt, and then hydrogenating said metal salt.

The hydroxylamine disulfonic acid alkali metal salts to be used as starting materials for the reaction of the present invention are compounds exemplified by hydroxylamine disulfonic acid sodium salt, which can be easily obtained by reacting sodium bisulfite with sodium nitrite in an aqueous solution, according to a known synthetic method of hydroxylamine salts ($NH_2OH \cdot A$, wherein A is an acid such as $H_2SO_4$, $H_3PO_4$, HCl, etc.). It is therefore possible to use the compound generated during the production of hydroxylamine as it is as the starting material for the present invention. [Translator's note: we believe that the sentence refers to the use of the reaction mixture.] It is also possible to isolate the corresponding product from the aqueous solution of said reaction, and then use it as the starting material for the present invention. Also, an aqueous solution of the hydroxylamine disulfonic acid alkali metal salt can be directly used for the reaction of the present invention. Sodium salt is the typical example of the alkali metal salt, but it is also possible to use potassium salt and lithium salt.

In the reaction of the present invention, a halogen compound represented by RX, such as an alkylating agent, is reacted with the aforementioned hydroxylamine disulfonic acid alkali metal salt in the presence of an aqueous medium.

Preferable examples for the substituent R of the aforementioned halogen compounds RX are a $C_{1-6}$ alkyl group such as methyl group, ethyl group, propyl group, butyl group, pentyl group, and hexyl group; $C_{1-6}$ alkenyl group such as aryl group, propenyl group and butenyl group; $C_{1-6}$ alkynyl group such as acetylene group, propynyl group, and butynyl group; aryl group such as phenyl group, naphthyl group, and anthranil group; aralkyl group such as benzyl group and phenethyl group; carboxyl group; ester group such as ethoxy carbonyl group; and ether group such as ethoxy ethyl group; where each of which can be substituted. The use of a $C_{1-6}$ alkyl group and carboxyl group is the most preferred. Examples of the substituents which may substitute R are alkyl group, alkoxy group, aryl group, aralkyl group, halogen atom, halogen-containing alkyl group, etc.

X is a halogen atom such as chlorine, bromine, fluorine, and iodine. The use of chlorine is preferred.

Reaction temperature and reaction time of the reaction according to formula (1) are not particularly limited, but the reaction is generally carried out for about 1 to 20 hours at a temperature of about 0 to 100°C, preferably 10 to 80°C. However, the reaction time should be suitably adjusted in consideration of the amount of the reaction material. When the halogen compound represented by RX is gaseous at the reaction temperature, the reaction is preferably carried out by using a pressure-resistant container such as an autoclave as the reactor.

The O-substituted hydroxylamine disulfonic acid alkali metal salts produced according to the reaction of formula (1) can be converted into the desired O-substituted hydroxylamine compounds by hydrogenation. The hydrogenation can be carried out by heating the O-substituted hydroxylamine disulfonic acid alkali metal salts in an aqueous medium in the presence of a strong acid such as sulfuric acid for hydrogenation, and then neutralizing the same with a base such as sodium hydroxide. The hydrogenation can proceed via reactions represented by the following formula (2) and (3), for example:

$$(MSO_3)_2NOR + H_2O \quad (H_2NOR)_2 \cdot H_2SO_4 + H_2SO_4 \qquad \cdots (2)$$

$$(H_2NOR)_2 \cdot H_2SO_4 + H_2SO_4 + NaOH \quad H_2NOR + Na_2SO_4 \quad \cdots (3)$$

The obtained O-substituted hydroxylamine compounds can be isolated as hydrochloride salts by addition of hydrochloric acid, for example.

[Examples]

[Example 1]

500mL of an aqueous solution of <u>hydroxylamine disulfonic acid sodium salt</u> (2 mols/L) and 80g of 50% aqueous sodium hydroxide solution were put into a pressure-resistant container. 50.5g of methyl chloride were given into another pressure-resistant container, and the two pressure-resistant containers were connected to each other so that the contents of each container could inter-circulate, and then the contents were mixed. The contents were stirred and mixed for 2 hours by maintaining the temperature inside the pressure-resistant containers at 50°C. After the pressure decrease inside the pressure-resistant containers (indicating the termination of the reaction), the pressure-resistant containers were opened, and the reaction liquid (aqueous solution of the obtained O-methyl hydroxylamine disulfonic acid sodium salt) was removed.

9.8g of concentrated sulfuric acid were added to the reaction liquid, and the mixture was heated at about 90°C for 24 hours under normal pressure, and then the mixture was hydrogenated to eliminate the sulfonic acid groups from the reaction product. After adding 330g of 50% aqueous sodium hydroxide solution to the hydrogenated liquid for neutralization, non-volatile contents such as salts contained in the neutralized liquid were removed by using a strip column. After that 66g (yield: 80%) of O-methyl hydroxylamine (boiling point: approx. 49°C) was obtained by distilling the eluents of the column.

171.7g of 15% hydrochloric acid were added to 33g of the obtained O-methyl hydroxylamine affording a 30% aqueous solution of O-methyl hydroxylamine hydrochloride.

[Example 2]

500mL of an aqueous solution of <u>hydroxylamine disulfonic acid sodium</u> (2 mols/L) and 80g of 50% aqueous sodium hydroxide solution were put into a beaker, and stirred for 24 hours at 50°C after adding 94.5g of chloroacetic acid. When the pH of the reaction liquid decreased to 9 or less and the termination of the reaction was confirmed, 9.8g of concentrated sulfuric acid were added to the reaction liquid, and the mixture was heated for 24 hours at approx. 90°C under normal pressure to hydrogenate the reaction product (O-carboxyl hydroxylamine disulfonic acid sodium salt) and cleave off its sulfonic acid groups. 50% aqueous sodium hydroxide solution was gradually added to the hydrogenated solution until the pH became around 7 for neutralization, and then the obtained amino-oxy acetic acid was extracted by using 700mL of ethyl acetate.
The ethyl acetate was removed from the extract under reduced pressure, and then 104g of 37% hydrochloric acid were added to the residue. The mixture was heated to 70°C to dissolve the residue. The residue was recrystallized at room temperature, and a crystal in which one molecule of hydrochloric acid was coordinated to two molecules of amino-oxy acetic acid was obtained (yield: 107g (98%)).

[Effect of the invention]

According to the production method of the present invention, it has become possible to produce O-substituted hydroxylamine compounds at a high yield by using industrially available hydroxylamine disulfonic acid alkali metal salt as the starting material.

**Claims**

1. Method for the production of O-substituted hydroxylamine compounds comprising:

   a process for obtaining O-substituted hydroxylamine disulfonic acid alkali metal salts by reacting a hydroxylamine disulfonic acid alkali metal salt with a halide according to the following formula (1):

   $$(MSO_3)_2NOH + RX \quad (MSO_3)_2NOR + HX \quad \cdots (1)$$

   [wherein M is an alkali metal atom; R is an alkyl group, alkenyl group, alkynyl group, aryl group, aralkyl group, carboxyl group, ester group, or ether group, where each group may have a substituent; and X is a halogen atom], and
   a process for hydrogenating the obtained O-substituted hydroxylamine disulfonic acid alkyl metal salt.

2. Method for the production of O-substituted hydroxylamine compounds according to Claim 1, wherein M is a sodium atom.

3. Method for the production of O-substituted hydroxylamine compounds according to Claim 1 or 2, wherein R is a $C_{1-6}$ alkyl group or carboxyl group.

## INTERNATIONAL SEARCH REPORT

| International application No. |
| --- |
| PCT/JP03/11086 |

**A. CLASSIFICATION OF SUBJECT MATTER**
Int.Cl$^7$ C07C239/20

According to International Patent Classification (IPC) or to both national classification and IPC

**B. FIELDS SEARCHED**

Minimum documentation searched (classification system followed by classification symbols)
Int.Cl$^7$ C07C239/20

Documentation searched other than minimum documentation to the extent that such documents are included in the fields searched

Electronic data base consulted during the international search (name of data base and, where practicable, search terms used)
CAPLUS(STN), CAOLD(STN), REGISTRY(STN)

**C. DOCUMENTS CONSIDERED TO BE RELEVANT**

| Category* | Citation of document, with indication, where appropriate, of the relevant passages | Relevant to claim No. |
| --- | --- | --- |
| A | JP 4-368360 A (Mitsui Petrochemical Industries, Ltd.), 21 December, 1992 (21.12.92), (Family: none) | 1-3 |
| A | JP 2-32048 A (Mitsui Petrochemical Industries, Ltd.), 01 February, 1990 (01.02.90), (Family: none) | 1-3 |

☐ Further documents are listed in the continuation of Box C.    ☐ See patent family annex.

| | |
| --- | --- |
| * Special categories of cited documents: <br> "A" document defining the general state of the art which is not considered to be of particular relevance <br> "E" earlier document but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |
| Date of the actual completion of the international search <br> 02 October 2003 (02.10.03) | Date of mailing of the international search report <br> 21 October, 2003 (21.10.03) |
| Name and mailing address of the ISA/ <br> Japanese Patent Office | Authorized officer |
| Facsimile No. | Telephone No. |

Form PCT/ISA/210 (second sheet) (July 1998)